# EUROPEAN PATENT APPLICATION

(11) **EP 4 796 035 A1**
(43) Date of publication of application: **26.08.2026**
(21) Application number: 24879574.2
(22) Date of filing: 03.10.2024
(51) Int. Cl.: A61B 5/256, A61B 5/291

(54) **BRAIN WAVE MEASURING DEVICE AND BRAIN WAVE MEASURING METHOD**

(30) Priority: 20.10.2023 JP 2023180770
(71) Applicant: Sumitomo Bakelite Co.Ltd., Shinagawa-ku Tokyo 140-0002 (JP)
(72) Inventor: ISHIKAWA, Eiji, Tokyo 140-0002 (JP)
(74) Representative: Ter Meer Steinmeister & Partner
(86) International application number: PCT/JP2024/035399
(87) International publication number: WO 2025/084148

(57) **Abstract**

A brain wave measuring device 10 including: a support member 20 which is disposed along a head 99 of a subject; an electrode unit 30 which is attached to the support member 20 and acquires a brain wave signal by coming into contact with a measured site of the subject; and an assistance member (attachment portion 70) which assists in positioning the support member 20 on the head 99, in which the support member 20 is configured with a non-stretchable member having a ribbon shape or a linear shape, and an elastic body (spring 61) having stretchability is provided between the support member 20 and an attachment portion to a human body (ear).

## Description

### TECHNICAL FIELD

The present invention relates to a brain wave measuring device and a brain wave measuring method.

### BACKGROUND ART

Various developments have been made in a brain wave measuring device which measures brain waves. As a kind of this technology, for example, a biological signal measuring device has been known, which includes a support which is made of a shape memory material and which is a head band mounted on the head of a user, and a vital sensor which is attached to the support and acquires a biological signal of the user (see, for example, Patent Document 1). According to the technology of Patent Document 1, in a case of measuring a biological signal, the support can be easily restored to a shape that matches the body shape of the user in which the shape of the support is stored in advance.

### RELATED DOCUMENT

### PATENT DOCUMENT

Patent Document 1: Japanese Patent No. 5900167

### SUMMARY OF THE INVENTION

### TECHNICAL PROBLEM

Meanwhile, in the measurement of the brain waves, it is important how to fit an electrode to a head shape in order to bring the electrode into contact with a scalp. However, the head shape varies widely, and a corresponding technology has been required. In addition, in a case where the electrode is brought into contact with the scalp, in a case where a force with which the electrode is pressed against the scalp is small, the subject does not feel pain or the like, but the brain wave acquisition may be unstable. In a case where the force with which the electrode is pressed against the scalp is large, the brain wave acquisition is stable, but the subject may easily feel pain or the like.

The present invention has been made in view of such a situation, and an object of the present invention is to provide a technology that enables, in brain wave measurement, an electrode to contact a scalp with an appropriate force when mounting a brain wave measuring device, particularly the electrode, into appropriate contact with the scalp.

### SOLUTION TO PROBLEM

According to the present invention, the following technologies are provided.
(1) A brain wave measuring device including:
   a support member which is disposed along a head of a subject;
   an electrode unit which is attached to the support member and acquires a brain wave signal by coming into contact with a measured site of the subject; and
   an assistance member which assists in positioning the support member on the head,
   in which the support member is configured with a non-stretchable member having a ribbon shape or a linear shape, and
   an elastic body having stretchability is provided between the support member and an attachment portion to a human body.
(2) The brain wave measuring device according to (1),
   in which the elastic body is a tension display unit that displays a tension acting on the support member in a recognizable manner.
(3) The brain wave measuring device according to (2), further including:
   a tension appropriateness display unit that displays whether or not the tension is within an appropriate range in a recognizable manner.
(4) The brain wave measuring device according to any one of (1) to (3),
   in which, in a case where the support member is configured with a member having a ribbon shape, the support member has a film member that is provided between adjacent electrode units.
(5) The brain wave measuring device according to (4),
   in which a Poisson's ratio of a material constituting the film member is equal to or more than 0.15 and equal to or less than 0.4.
(6) The brain wave measuring device according to (4),
   in which a product of an elastic modulus and a thickness of a material constituting the film member is equal to or more than 0.4 and equal to or less than 9.1.
(7) The brain wave measuring device according to any one of (1) to (3),
   in which in a case where the support member is configured with a member having a linear shape, the member having the linear shape is provided between electrode units.
(8) The brain wave measuring device according to (7),
   in which the member having the linear shape includes at least two members that extend to be spaced apart from each other, and the electrode units are provided between the two members.
(9) The brain wave measuring device according to any one of (1) to (8),
   in which the electrode unit includes a base portion, a plurality of protruding portions provided on the base portion, and an electrode portion that is provided on the protruding portions and that comes into contact with the head.
(10) The brain wave measuring device according to (9),
   in which the protruding portions are elastic members.
(11) The brain wave measuring device according to any one of (1) to (10),
   in which the support member is provided at an end portion of the support member, is attached to an ear or a jaw, and assists disposition of the support member to a head shape.
(12) A brain wave measuring method including:
   mounting the brain wave measuring device according to any one of (1) to (11) on a head of a subject to measure a brain wave.

### ADVANTAGEOUS EFFECTS OF INVENTION

According to the present invention, in brain wave measurement, it is possible to provide a technology that enables an electrode to contact a scalp with an appropriate force when mounting a brain wave measuring device, particularly the electrode, into appropriate contact with the scalp.

### BRIEF DESCRIPTION OF THE DRAWINGS

[FIG. 1] A schematic view showing a state where a brain wave measuring device according to a first embodiment is mounted on a head.
[FIG. 2] A schematic view showing a support member and an attachment portion according to the first embodiment.
[FIG. 3] A schematic view showing a tension display unit according to the first embodiment.
[FIG. 4] A diagram illustrating, by modeling, a force (electrode pressing force F on the head) acting on the head in a case where the brain wave measuring device is mounted according to the first embodiment.
[FIG. 5] A diagram illustrating a capstan principle (equation) used for the modeling according to the first embodiment.
[FIG. 6] A cross-sectional view of an electrode unit in a state of being mounted on the support member according to the first embodiment.
[FIG. 7] A schematic view showing a state where a brain wave measuring device according to a second embodiment is mounted on a head.
[FIG. 8] A schematic view showing a support member and an attachment portion according to the second embodiment.
[FIG. 9] A cross-sectional view of an electrode unit in a state of being mounted on the support member according to the second embodiment.
[FIGS. 10A and 10B] Diagrams schematically showing an adjustment unit according to the second embodiment.
[FIG. 11] A plan view of a brain wave measuring device according to a third embodiment.
[FIG. 12] A front view of the brain wave measuring device according to the third embodiment.
[FIG. 13] A plan view focusing on an attachment aspect of a support member for two electrode units at electrode positions Cz and C3 according to the third embodiment.
[FIG. 14] A cross-sectional view of an electrode unit in a state of being mounted on the support member according to the third embodiment.
[FIG. 15] A graph showing theoretical values (calculated values) and measured values of an electrode pressing force F according to Examples.

### DESCRIPTION OF EMBODIMENTS

Hereinafter, embodiments of the present invention will be described with reference to the drawings. In the present embodiment, a brain wave measuring device which has an electrode unit and is mounted on a head of a subject to acquire brain waves, and a brain wave measuring method using the brain wave measuring device will be described.

### <First Embodiment>

### <Schematic Structure of Brain Wave Measuring Device 10>

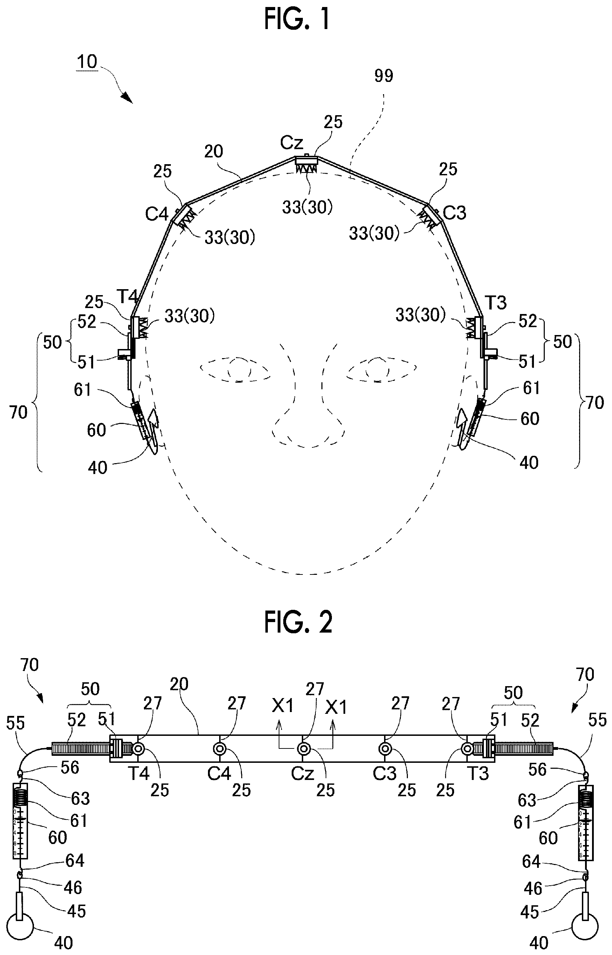

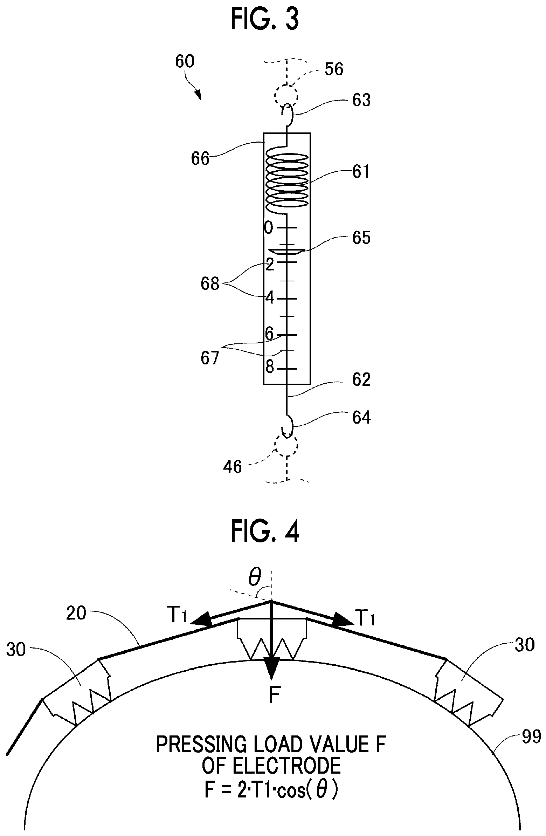

FIG. 1 is a schematic view of a state where a brain wave measuring device 10 is mounted on a head 99 of a person, as viewed from the front. FIG. 2 is a view showing a support member 20 and an attachment portion 70, which support an electrode unit 30. In the present embodiment, the brain wave measuring device 10 which measures brain waves at electrode positions Cz, C3, C4, T3, and T4 (international 10-20 system) is described. FIG. 3 is a schematic view showing a tension display unit 60.

The brain wave measuring device 10 is mounted on the head 99 of the person, detects brain waves as a potential fluctuation from a living body, and outputs the detected brain waves to a brain wave display device (not shown in the drawings). The brain wave display device acquires the brain waves detected by the brain wave measuring device 10, and performs monitor display, data storage, and a well-known brain wave analysis process (measurement process).

The brain wave measuring device 10 has a plurality of electrode units 30 which come into contact with a measured site (that is, a head 99) of a subject to acquire brain waves as a biological signal, a support member 20 in a long sheet shape (film base material), which supports the electrode units 30 by attachment, and an attachment portion 70 which fixes the support member 20 to the head 99. In the present embodiment, the brain wave measuring device 10 is fixed to the head 99 by mounting the attachment portion 70 on the ear of the subject. The attachment portion 70 includes a tension display unit 60 having a tension display function of displaying a tension acting on the support member 20 in a recognizable manner.

The support member 20 is configured to be bendable to some extent at least in a thickness direction. In addition, a member (so-called hard member such as a helmet) which is provided in advance with a shape by surrounding the support member 20 is not provided. In a state where the support member 20 and the like are not mounted on the head 99, the support member 20 and the like can be arranged in a flat state without being applied by an external force; and in a case of being mounted on the head 99, the shape of the support member 20 and the like is mainly changed along the shape of the head 99 by gravity. Since both end portions of the electrode unit 30 may be in a state of being lifted from the head 99 due to influence of head hair or the like, the attachment portion 70 is used for supporting the follow-up to the shape of the head 99.

The support member 20 is provided with a recessed snap button 25 for fixing the electrode unit 30, and a protruding snap button 35 of the electrode unit 30 is fitted into the recessed snap button 25. In addition, in the support member 20, a position where the recessed snap buttons 25 are provided in the left-right direction coincides with a bent portion 27 where the support member 20 is bent. In a case where the support member 20 is sufficiently thin, the bent portion 27 is not necessary, but by providing the bent portion 27, the support member 20 between the electrode units 30 can be made linear.

The attachment portion 70 includes an ear attachment portion 40, an adjustment unit 50, and the tension display unit 60, and these are connected by a member (here, cord 45, 55) that does not extend.

In a case where the brain wave measuring device 10 is mounted on the head 99, the position of the electrode units 30 is generally a vertex of a polygonal shape, and the shape is provided between the electrode units 30 by the support member 20. That is, the support member 20 is bent at a portion (bent portion 27) that coincides with a portion to which the electrode unit 30 is attached.

### <Electrode Pressing Force F on Head>

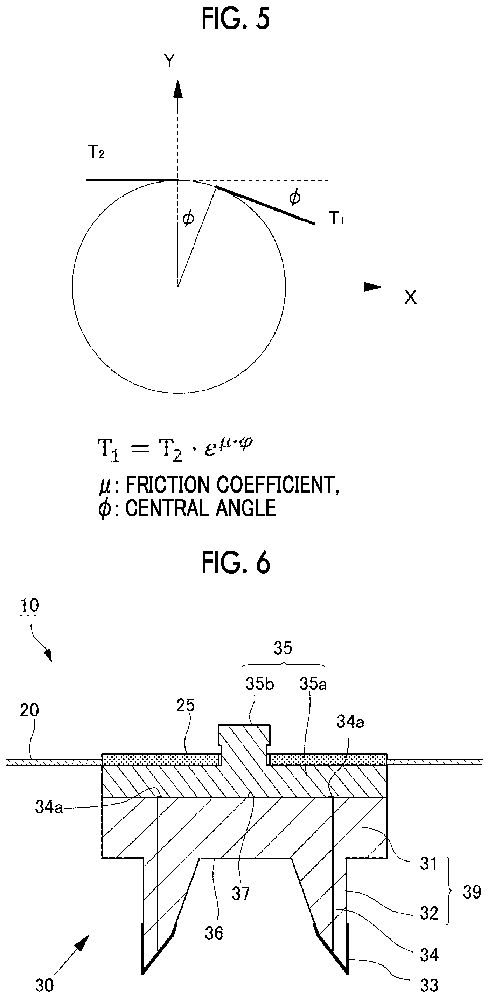

Here, features of the present embodiment will be described with reference to FIGS. 4 and 5. FIG. 4 is a diagram illustrating, by modeling, a force (electrode pressing force F on the head) acting on the head 99 in a case where the brain wave measuring device 10 is mounted. FIG. 5 is a diagram illustrating a capstan principle (equation) used for the modeling.

As shown in FIG. 4, in a state where the brain wave measuring device 10 is mounted on the head 99, a polygonal shape is formed with the plurality of electrode units 30 as vertices and the support member 20 connecting the electrode units 30. In this case, assuming that the electrode unit 30 is pressed against the head 99 by a tension T₁ of the support member 20, a force (electrode pressing force F) for pressing the electrode unit 30 against the head 99 can be represented by the following equation 1. $F = 2 ⋅ {T}_{1} ⋅ cos \left(θ\right)$

That is, a difference in θ caused by individual difference in unevenness of the head appears as the difference in the electrode pressing force F.

Furthermore, the minimum curvature of an inscribed circle is obtained from a height and an interval of the electrode units 30. In addition, the maximum curvature is obtained from the capstan principle with the electrode pressing force F required for the electrode unit 30. Since the electrode pressing force F on the head 99 follows the capstan equation, the force is larger on a temporal region than on a vertex region.

Here, it is assumed that the support member 20 (film base material) is approximately in a state of being wound around the head 99. Since there is friction between the head 99 and the support member 20, it is considered that the tension of each electrode unit 30 follows the capstan equation shown in the following equation 2. ${T}_{1} = {T}_{2} ⋅ {e}^{∧} \left(μ⋅φ\right)$
µ: Friction coefficient
φ: Central angle
T₁: Tension of the other action point rotated by the central angle φ with the one action point as a point on the Y axis in a case where the head 99 is assumed to be an outer periphery of a circle and the central angle corresponding to the arc around which the support member 20 is wound around the head 99 is defined as φ
T₂: Tension of the action point in a case where the head 99 is assumed to be an outer periphery of a circle and the central angle corresponding to the arc around which the support member 20 is wound around the head is defined as φ, and the one action point is a point on the Y axis

A relational expression between a tension T1 applied to the end portion of the support member 20 (film base material) from Equation 1 and Equation 2 and a load (electrode pressing force F) applied to the head 99 by the electrode unit 30 can be grasped.

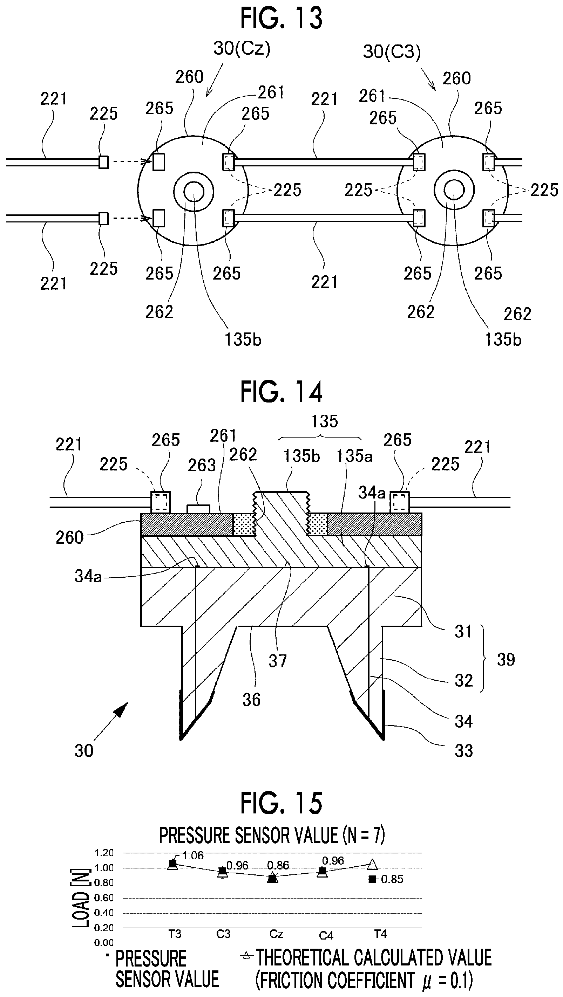

The load value calculated from the above-described theoretical calculation and a load value actually measured will be shown in Examples (see FIG. 15) described later.

### <Electrode Unit 30>

The electrode unit 30 is attachably and detachably provided only at a portion required for the brain wave measurement. Attachment positions of the electrode units 30 correspond to, for example, the positions of T3, C3, Cz, C4, and T4 in the international 10-20 system, and are arranged to be symmetrical in front view as shown in FIG. 1.

FIG. 6 shows a cross-sectional view of the electrode unit 30 in a state of being attached to the recessed snap button 25 of the support member 20. The electrode unit 30 is configured as a button electrode and has the protruding snap button 35 provided as a connection terminal, and the protruding snap button 35 is detachably attached to the recessed snap button 25 provided at a predetermined position of the support member 20. That is, the recessed snap button 25 functions as a mounting unit for attaching the electrode unit 30 by the fitting structure.

The electrode unit 30 may be configured with a conductive metal as a whole, or may have a configuration in which a conductive member is provided on a surface of the electrode unit based on a rubber-like elastic member. Hereinafter, the configuration based on the rubber-like elastic member will be exemplified.

The detailed structure and material of the electrode unit 30 will be described later, but the electrode unit 30 according to the present embodiment is an electrode (a dry electrode) which does not use a so-called paste for a brain wave electrode in order to ensure conductivity. However, a method of forming a gel on the dry electrode tip (tip of the protrusion portion 32) and immersing the gel in a wetting liquid or the like to secure moisture may be adopted. In addition, a method of wetting the dry electrode tip with a conductive auxiliary liquid in which a small amount of an electrolyte such as salt is mixed with a low-viscosity liquid such as a lotion, instead of the paste or a grease, may be adopted. That is, the dry electrode according to the present embodiment is not limited to the completely dry electrode, but is intended not to use an auxiliary agent such as a paste in which significant staining remain.

The electrode unit 30 has a base portion 31 having a columnar shape, a protrusion portion 32 which is provided integrally with one end of the base portion 31 (here, a base portion lower surface 36), a conductive contact portion 33, a signal line portion 34, and a protruding snap button 35 which is provided at the other end of the base portion 31 (here, a base portion upper surface 37). Hereinafter, the base portion 31 and the protrusion portion 32 will be referred to as an electrode portion main body 39 for convenience.

The electrode portion main body 39 is integrally provided by a rubber-like elastic member. The specific material of the elastic member will be described later. The electrode portion main body 39 (that is, the base portion 31 and the protrusion portion 32) is not limited to the configuration of being integrally provided, and may be configured by assembling those provided separately by an adhesive or a fitting structure.

### <Shape of Base Portion 31 and Protrusion Portion 32>

The base portion 31 has a substantially columnar shape. A plurality of substantially conical protrusion portions 32 which protrude in a downward direction in the drawing are provided on the circular base portion lower surface 36 on one end side of the base portion 31 (the lower side in the drawing). It is sufficient that the base portion 31 have the columnar shape, and a cross section thereof may have a shape other than a circle, such as a polygonal shape. The shape of the protrusion portion 32 is not limited to the conical shape, and various shapes of a pyramid such as a triangular pyramid, a columnar shape, and the like may be adopted.

The conductive contact portion 33 is provided on at least a distal end side surface of the protrusion portion 32. The conductive contact portion 33 may be provided on the entire surface of the protrusion portion 32. The conductive contact portion 33 is formed in a thin film shape, and in a state where the conductive contact portion 33 is provided on the protrusion portion 32, the protrusion portion 32 with the conductive contact portion 33 can be regarded as having substantially the same shape as the shape of only the protrusion portion 32.

The outer diameter of the base portion 31 is, for example, 10 mm to 50 mm. A height (thickness) of the base portion 31 is, for example, 0.1 mm to 30 mm. A height of the protrusion portion 32 is, for example, 1 mm to 20 mm. The width (outer diameter of the root portion) of the protrusion portion 32 is, for example, 1 mm to 10 mm.

### <Material of Electrode Portion Main Body 39 (Base Portion 31 and Protrusion Portion 32)>

A material of the electrode portion main body 39 will be described. The electrode portion main body 39 may be made of a rubber-like elastic body as described above. Specifically, the rubber-like elastic body is a rubber or a thermoplastic elastomer (also simply referred to as "elastomer (TPE)"). Examples of the rubber include a silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), amide-based TPE (TPAE), and the like.

In a case where a material of the electrode portion main body 39 is silicone rubber, a rubber hardness A is, for example, 15 or more and 55 or less in a case where a type A durometer hardness of the surface of the electrode portion main body 39 is measured at 37°C in accordance with JIS K 6253 (1997) and is defined as the rubber hardness A.

Here, the above-described silicone rubber-based curable composition will be described.

The above-described silicone rubber can be formed of a cured product of the silicone rubber-based curable composition. A curing step of the silicone rubber-based curable composition is performed by heating (primary curing) the composition, for example, at 100°C to 250°C for 1 to 30 minutes and post-baking (secondary curing) the heated composition at 100°C to 200°C for 1 to 4 hours.

Insulating silicone rubber is silicone rubber not containing a conductive filler, and conductive silicone rubber is silicone rubber containing a conductive filler.

The silicone rubber-based curable composition according to the present embodiment may contain a vinyl group-containing organopolysiloxane (A). The vinyl group-containing organopolysiloxane (A) is a polymer contained in the silicone rubber-based curable composition according to the present embodiment as a main component.

The insulating silicone rubber-based curable composition and the conductive silicone rubber-based curable composition may contain the same kind of vinyl group-containing linear organopolysiloxane. It is sufficient that the same kind of vinyl group-containing linear organopolysiloxane includes at least a vinyl group having the same functional group and is linear, and it may have different vinyl group amounts or molecular weight distributions in the molecule, or different addition amounts thereof.

The insulating silicone rubber-based curable composition and the conductive silicone rubber-based curable composition may further contain different kinds of vinyl group-containing organopolysiloxanes.

The vinyl group-containing organopolysiloxane (A) can contain a vinyl group-containing linear organopolysiloxane (A1) having a linear structure.

The above-described vinyl group-containing linear organopolysiloxane (A1) has a linear structure and contains a vinyl group, in which the vinyl group functions as a crosslinking point during the curing.

The content of the vinyl group in the vinyl group-containing linear organopolysiloxane (A1) is not particularly limited. For example, the vinyl group in the vinyl group-containing linear organopolysiloxane (A1) has two or more vinyl groups in a molecule, and the content of the vinyl groups is preferably 15% by mole or less and more preferably 0.01% to 12% by mole. In a case where the content of the vinyl group is in the above range, the amount of the vinyl group in the vinyl group-containing linear organopolysiloxane (A1) can be optimized, and a network between the respective components, which will be described later, can be reliably formed. In the present embodiment, "to" means that the range includes numerical values as an upper limit value and a lower limit value.

In the present specification, the content of the vinyl group represents mol% of a vinyl group-containing siloxane unit with respect to 100 mol% of all units forming the vinyl group-containing linear organopolysiloxane (A1). Here, it is assumed that there may be one vinyl group for each vinyl group-containing siloxane unit.

In addition, a polymerization degree of the vinyl group-containing linear organopolysiloxane (A1) is not particularly limited, and is, for example, preferably in a range of approximately 1,000 to 10,000 and more preferably in a range of approximately 2,000 to 5,000. The polymerization degree can be obtained as, for example, a number-average polymerization degree (or number-average molecular weight) in terms of polystyrene in gel permeation chromatography (GPC) in which chloroform is used as an eluent.

Furthermore, a specific gravity of the vinyl group-containing linear organopolysiloxane (A1) is not particularly limited and is preferably in a range of approximately 0.9 to 1.1.

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

It is preferable that the vinyl group-containing linear organopolysiloxane (A1) has a structure represented by Formula (1).

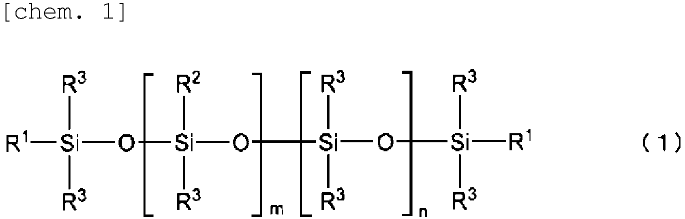

In Formula (1), R¹ is a substituted or unsubstituted alkyl group, alkenyl group, or aryl group having 1 to 10 carbon atoms, or a hydrocarbon group formed by the combination of these groups. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Among these, a vinyl group is preferable. Examples of the aryl group having 1 to 10 carbon atoms include a phenyl group.

In addition, R² represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R³ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group.

Further, examples of a substituent of R¹ and R² in Formula (1) include a methyl group and a vinyl group. Examples of a substituent of R³ include a methyl group.

In Formula (1), a plurality of R¹'s may be independent from each other and may be the same as or different from each other. Further, this shall apply to R² and R³.

Furthermore, m and n are the number of repeating units constituting the vinyl group-containing linear organopolysiloxane (A1) represented by Formula (1), m is an integer of 0 to 2,000, and n is an integer of 1,000 to 10,000, where m is preferably 0 to 1,000 and n is preferably 2,000 to 5,000.

Examples of specific structures of the vinyl group-containing linear organopolysiloxane (A1) represented by Formula (1) include a structure represented by Formula (1-1).

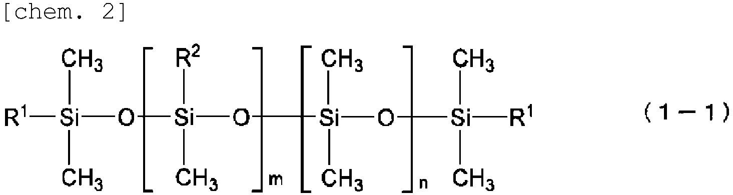

In Formula (1-1), R¹ and R² are each independently a methyl group or a vinyl group, and at least one of R¹ and R² is the vinyl group.

Furthermore, as the vinyl group-containing linear organopolysiloxane (A1), it is preferable to contain a first vinyl group-containing linear organopolysiloxane (A1-1) having two or more vinyl groups in the molecule, in which the content of the vinyl group is 0.4 mol% or less, and a second vinyl group-containing linear organopolysiloxane (A1-2) in which the content of the vinyl group is 0.5 to 15 mol%. Combining the first vinyl group-containing linear organopolysiloxane (A1-1) having the general vinyl group content and the second vinyl group-containing linear organopolysiloxane (A1-2) having a high vinyl group content as raw rubber that is a raw material of the silicone rubber enables vinyl groups to be unevenly distributed, which makes it possible to more effectively form variations in the crosslinking density in the crosslinked network of the silicone rubber. As a result, the tear strength of the silicone rubber can be more effectively improved.

Specifically, as the vinyl group-containing linear organopolysiloxane (A1), for example, the first vinyl group-containing linear organopolysiloxane (A1-1) having two or more of a unit in which R¹ in Formula (1-1) represents a vinyl group and/or a unit in which R² in Formula (1-1) represents a vinyl group in the molecule and the content is 0.4 mol% or lower, and the second vinyl group-containing linear organopolysiloxane (A1-2) including 0.5 to 15 mol% of a unit in which R¹ in Formula (1-1) represents a vinyl group and/or a unit in which R² in Formula (1-1) represents a vinyl group are preferably used.

In addition, in the first vinyl group-containing linear organopolysiloxane (A1-1), the content of the vinyl group is preferably 0.01 to 0.2 mol%. In addition, the second vinyl group-containing linear organopolysiloxane (A1-2) preferably has a vinyl group content of 0.8% to 12% by mole.

Furthermore, in a case where the first vinyl group-containing linear organopolysiloxane (A1-1) and the second vinyl group-containing linear organopolysiloxane (A1-2) are blended in combination, a ratio between (A1-1) and (A1-2) is not particularly limited. However, a weight ratio (A1-1):(A1-2) is preferably 50:50 to 95:5, and more preferably 80:20 to 90:10.

As each of the first vinyl group-containing linear organopolysiloxane (A1-1) and the second vinyl group-containing linear organopolysiloxane (A1-2), only one kind may be used or two or more kinds may be used in combination.

The vinyl group-containing organopolysiloxane (A) may contain a vinyl group-containing branched organopolysiloxane (A2) having a branched structure.

### <<Organohydrogen Polysiloxane (B)>>

The silicone rubber-based curable composition of the present embodiment may contain a crosslinking agent. The crosslinking agent may contain an organohydrogen polysiloxane (B).

The organohydrogen polysiloxane (B) is classified into a linear organohydrogen polysiloxane (B1) having a linear structure and a branched organohydrogen polysiloxane (B2) having a branched structure, and can include either or both of these.

The insulating silicone rubber-based curable composition and the conductive silicone rubber-based curable composition may contain the same kind of crosslinking agent. It is sufficient that the same kind of crosslinking agent has at least a common structure such as a linear structure or a branched structure, and it may have different molecular weight distributions in the molecule, different functional groups, or different addition amounts thereof.

The insulating silicone rubber-based curable composition and the conductive silicone rubber-based curable composition may further contain different types of crosslinking agents.

The linear organohydrogen polysiloxane (B1) is a polymer that has a linear structure and a structure (≡Si-H) in which hydrogen is directly bonded to Si, and has a hydrosilylation reaction with vinyl groups contained in the components blended with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably less than or equal to 20,000 and more preferably greater than or equal to 1,000 and less than or equal to 10,000.

The weight-average molecular weight of the linear organohydrogen polysiloxane (B1) can be measured as a polystyrene-equivalent value by gel permeation chromatography (GPC) using chloroform as an eluent.

In addition, it is preferable that the linear organohydrogen polysiloxane (B1) generally does not contain vinyl groups. As a result, the crosslinking reaction in the molecule of the linear organohydrogen polysiloxane (B1) can be reliably prevented from progressing.

As such a linear organohydrogen polysiloxane (B1), for example, it is preferable to use an organohydrogen polysiloxane having a structure represented by Formula (2).

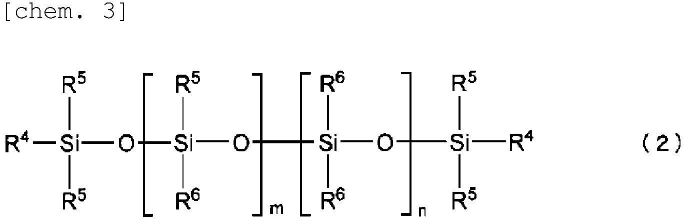

In Formula (2), R⁴ is a substituted or unsubstituted alkyl group, alkenyl group, or aryl group having 1 to 10 carbon atoms, a hydrocarbon group formed by the combination of these groups, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R⁵ is a substituted or unsubstituted alkyl group, alkenyl group, or aryl group having 1 to 10 carbon atoms, a hydrocarbon group formed by the combination of these groups, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

Furthermore, in Formula (2), a plurality of R⁴'s are independent of each other, and may be the same as or different from each other. The same applies to R⁵. In this case, at least two or more among a plurality of R⁴'s and a plurality of R⁵'s represent a hydride group.

In addition, R⁶ is a substituted or unsubstituted alkyl group or aryl group having 1 to 8 carbon atoms, or a hydrocarbon group formed by the combination of these groups. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group. A plurality of R⁶'s may be independent from each other and may be the same as or different from each other.

Furthermore, examples of the substituent of R⁴, R⁵, and R⁶ in Formula (2) include a methyl group and a vinyl group, and the methyl group is preferable from the viewpoint of preventing the crosslinking reaction within the molecule.

Furthermore, m and n each independently represent the number of repeating units forming the linear organohydrogen polysiloxane (B1) represented by Formula (2), and m represents an integer of 2 to 150 and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

Since the branched organohydrogen polysiloxane (B2) has a branched structure, it is a component that largely contributes to formation of a structure with variations in crosslinking density in the silicone rubber system by forming a region having a high crosslinking density. In addition, as in the above-described linear organohydrogen polysiloxane (B1), the branched organohydrogen polysiloxane (B2) is a polymer that has a structure in which hydrogen is directly bonded to Si (≡Si-H), and that undergoes a hydrosilylation reaction of the vinyl group in the vinyl group-containing organopolysiloxane (A) and a vinyl group in a component blended in the silicone rubber-based curable composition to crosslink the components.

In addition, a specific gravity of the branched organohydrogen polysiloxane (B2) is in a range of 0.9 to 0.95.

It is preferable that the branched organohydrogen polysiloxane (B2) contains no vinyl group in general. As a result, the crosslinking reaction in the molecule of the branched organohydrogen polysiloxane (B2) can be reliably prevented from progressing.

In addition, it is preferable that the branched organohydrogen polysiloxane (B2) is represented by Average compositional formula (c) below.

Average compositional formula (c) (Hₐ(R⁷)₃₋ₐSiO_{1/2})ₘ(SiO_{4/2})ₙ

(In Formula (c), R⁷ is a monovalent organic group, a is an integer in a range of 1 to 3, m is the number of Hₐ(R⁷)₃₋ₐSiO_{1/2} units, and n is the number of SiO_{4/2} units)
In Formula (c), R⁷ is a monovalent organic group, preferably a substituted or unsubstituted alkyl group or aryl group having 1 to 10 carbon atoms, or a hydrocarbon group formed by the combination of these groups. Examples of the alkyl group having 1 to 10 carbon atoms include a methyl group, an ethyl group, and a propyl group. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3 and preferably 1.

In addition, in Formula (c), m is the number of Hₐ(R⁷)₃₋ₐSiO_{1/2} units, and n is the number of SiO_{4/2} units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si is in a range of 1.8 to 2.1 in the linear organohydrogen polysiloxane (B1) and is in a range of 0.8 to 1.7 in the branched organohydrogen polysiloxane (B2).

Since the branched organohydrogen polysiloxane (B2) has a branched structure, an amount of residues is 5% or more, for example, after heating to 1,000°C at a temperature increase rate of 10 °C/min in a nitrogen atmosphere. On the other hand, since the linear organohydrogen polysiloxane (B1) is linear, an amount of residues after the heating under the above-described conditions is substantially 0.

In addition, specific examples of the branched organohydrogen polysiloxane (B2) include an organohydrogen polysiloxane having a structure represented by Formula (3).

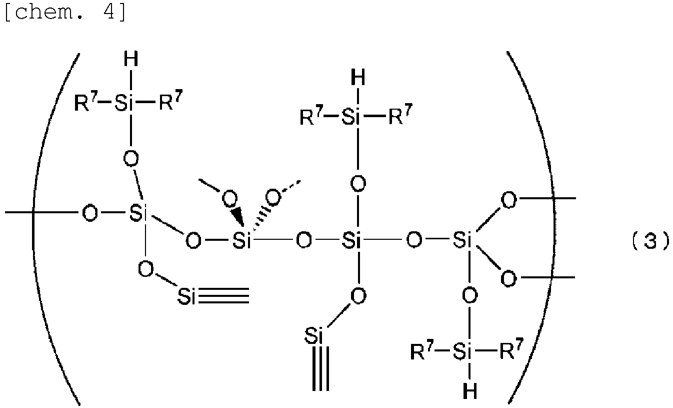

In Formula (3), R⁷ is a substituted or unsubstituted alkyl group or aryl group having 1 to 8 carbon atoms, or a hydrocarbon group formed by the combination of these groups, or a hydrogen atom. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group. Examples of the substituent of R⁷ include a methyl group.

Furthermore, in Formula (3), a plurality of R⁷'s are independent of each other, and may be the same as or different from each other.

In addition, in Formula (3), "-O-Si≡" represents that Si has a branched structure which spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), an amount of hydrogen atoms directly bonded to Si (hydride groups) is not particularly limited. However, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

### <<Silica Particles (C)>>

The silicone rubber-based curable composition of the present embodiment contains a non-conductive filler. The non-conductive filler may include silica particles (C) as necessary. As a result, hardness and mechanical strength of the elastomer can be improved.

The insulating silicone rubber-based curable composition and the conductive silicone rubber-based curable composition may contain the same type of non-conductive fillers. It is sufficient that the same kind of non-conductive filler may have at least a common constituent material, and the particle diameter, the specific surface area, the surface treatment agent, or the addition amounts may be different.

The insulating silicone rubber-based curable composition and the conductive silicone rubber-based curable composition may further contain different types of silane coupling agents.

The silica particles (C) are not particularly limited. For example, fumed silica, sintered silica, precipitated silica, or the like is used. These may be used alone or in combination of two or more thereof.

The specific surface area of the silica particles (C) measured using, for example, a BET method is, for example, preferably 50 to 400 m²/g and more preferably 100 to 400 m²/g. The average primary particle diameter of the silica particles (C) is, for example, preferably 1 to 100 nm and more preferably about 5 to 20 nm.

Using the silica particles (C) having the specific surface area and the average particle diameter in the above ranges makes it possible to improve the hardness and the mechanical strength of the formed silicone rubber and, particularly, to improve the tensile strength of the formed silicone rubber.

### <<Silane Coupling Agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The insulating silicone rubber-based curable composition and the conductive silicone rubber-based curable composition may contain the same kind of silane coupling agent. It is sufficient that the same kind of silane coupling agent has at least a common functional group, and it may have different functional groups in the molecule or different addition amounts thereof.

The insulating silicone rubber-based curable composition and the conductive silicone rubber-based curable composition may further contain different types of silane coupling agents.

In addition, the silane coupling agent (D) may include a silane coupling agent having a hydrophobic group. As a result, the hydrophobic group is added to a surface of the silica particles (C), and in the silicone rubber-based curable composition and in the silicone rubber, it is presumed that a cohesion force between the silica particles (C) decreases (cohesion through a hydrogen bond formed by a silanol group decreases), and thus a dispersibility of the silica particles (C) in the silicone rubber-based curable composition is improved. Consequently, an interface between the silica particles (C) and a rubber matrix enlarges, and a reinforcing effect of the silica particles (C) increases. Furthermore, it is presumed that, when the rubber matrix is deformed, a slidability of the silica particles (C) in the matrix is improved. By improving the dispersibility and slidability of the silica particles (C), a mechanical strength (for example, a tensile strength, a tear strength, or the like) of the silicone rubber by the silica particles (C) is improved.

Furthermore, the silane coupling agent (D) may include a silane coupling agent having a vinyl group. As a result, the vinyl group is introduced onto the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure) using this reaction, the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group can be used in combination.

Examples of the silane coupling agent (D) include a compound represented by the following Formula (4).

Yₙ-Si-(X)₄₋ₙ ... (4)

In Formula (4), n represents an integer of 1 to 3. Y represents any functional group selected from the group consisting of a hydrophobic group, a hydrophilic group, and a vinyl group, in a case where n represents 1, Y represents a hydrophobic group, and in a case where n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples thereof include a methyl group, an ethyl group, a propyl group, and a phenyl group; and among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be included as a functional group, but from the viewpoint of imparting hydrophobicity to the silane coupling agent (D), it is preferable that the hydrophilic group is not included.

Examples of the hydrolyzable group include an alkoxy group such as a methoxy group or an ethoxy group, a chloro group, a silazane group, and the like. Among these, in view of high reactivity with the silica particles (C), a silazane group is preferable. In view of structural characteristics of the coupling agent, the silane coupling agent having a silazane group as the hydrolyzable group has a structure including two structures represented by (Yₙ-Si-) in Formula (4).

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; a chlorosilane such as methyltrichlorosilane, dimethyldichlorosilane, trimethylchlorosilane, and phenyltrichlorosilane; and hexamethyldisilazane. Among these, a silane coupling agent having a trimethylsilyl group, which includes one or more selected from the group consisting of hexamethyldisilazane, trimethylchlorosilane, trimethylmethoxysilane, and trimethylethoxysilane, is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane; a chlorosilane such as vinyltrichlorosilane and vinylmethyldichlorosilane; and divinyltetramethyldisilazane. Among these, a silane coupling agent having a vinyl group-containing organosilyl group, which includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane, is preferable.

In addition, in a case where the silane coupling agent (D) includes two kinds including the silane coupling agent having a trimethylsilyl group and the silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited. However, for example, a weight ratio (D1):(D2) is 1:0.001 to 1:0.35, preferably 1:0.01 to 1:0.20, and more preferably 1:0.03 to 1:0.15. In a case where the ratio is set to be in the above-described numerical ranges, desired physical properties of silicone rubber can be obtained. Specifically, a balance between the dispersibility of silica in the rubber and the crosslinkability of the rubber can be achieved.

In the present embodiment, a lower limit value of a content of the silane coupling agent (D) is preferably 1 mass% or more, more preferably 3 mass% or more, and still more preferably 5 mass% or more with respect to 100 parts by weight of the total amount of the vinyl group-containing organopolysiloxane (A). In addition, an upper limit value of the content of the silane coupling agent (D) is preferably 100 mass% or less, more preferably 80 mass% or less, and still more preferably 40 mass% or less with respect to 100 parts by weight of the total amount of the vinyl group-containing organopolysiloxane (A).

By adjusting the content of the silane coupling agent (D) to be the above-described lower limit value or more, adhesiveness between a columnar portion containing an elastomer and a conductive resin layer can be improved. In addition, the improvement of the mechanical strength of the silicone rubber can be promoted. In addition, by adjusting the content of the silane coupling agent (D) to be the above-described upper limit value or less, the silicone rubber can have appropriate mechanical properties.

### <<Platinum or Platinum Compound (E)>>

The silicone rubber-based curable composition according to the present embodiment may contain a catalyst. The catalyst may include platinum or platinum compound (E). The platinum or platinum compound (E) is a catalyst component which functions as a catalyst during the curing. The addition amount of the platinum or platinum compound (E) is the catalytic amount.

The insulating silicone rubber-based curable composition and the conductive silicone rubber-based curable composition may contain the same type of catalysts. It is sufficient that the same kind of catalyst has at least a common constituent material, and it may have different compositions in the catalyst or different addition amounts thereof.

The insulating silicone rubber-based curable composition and the conductive silicone rubber-based curable composition may further contain different kinds of catalysts.

As the platinum or platinum compound (E), known platinum or platinum compounds can be used. Examples thereof include platinum black, silica or carbon black on which platinum is supported, chloroplatinic acid or an alcohol solution of platinic chloride, a complex salt of platinic chloride and olefin, a complex salt of platinic chloride and vinylsiloxane, and the like.

As the platinum or platinum compound (E), only one kind may be used alone or two or more kinds may be used in combination.

In the present embodiment, a content of the platinum or platinum compound (E) in the silicone rubber-based curable composition refers to the catalytic amount and can be appropriately set. Specifically, a content of platinum-group metal in units of weight is 0.01 to 1000 ppm, preferably 0.1 to 500 ppm, with respect to 100 parts by weight of the total amount of the vinyl group-containing organopolysiloxane (A), the silica particles (C), and the silane coupling agent (D).

By adjusting the content of the platinum or platinum compound (E) to be the above-described lower limit value or more, the silicone rubber-based curable composition can be cured at an appropriate rate. In addition, by adjusting the content of the platinum or platinum compound (E) to be the above-described upper limit value or less, a reduction in manufacturing costs can be promoted.

### <<Water (F)>>

In addition, the silicone rubber-based curable composition according to the present embodiment may contain water (F) in addition to the above-described components (A) to (E).

The water (F) is a component that functions as a dispersion medium for dispersing the respective components in the silicone rubber-based curable composition and contributes to the reaction between the silica particles (C) and the silane coupling agent (D). Therefore, in the silicone rubber, the silica particles (C) and the silane coupling agent (D) can be more reliably bonded to each other, and uniform properties can be exhibited as a whole.

### (Other Components)

Furthermore, the silicone rubber-based curable composition according to the present embodiment may further contain other components in addition to the above-described components (A) to (F). Examples of the other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, and mica; and an additive such as a reaction inhibitor, a dispersant, a pigment, a dye, an antistatic agent, an antioxidant, a flame retardant, and a thermal conductivity enhancing agent.

The conductive solution (conductive silicone rubber composition) according to the present embodiment contains the above-described conductive filler and a solvent, in addition to the above-described silicone rubber-based curable composition not containing the conductive filler.

As the above-described solvent, various well-known solvents can be used, and for example, a high boiling point solvent can be included. These may be used alone or in combination of two or more thereof.

Examples of the solvent include an aliphatic hydrocarbon such as pentane, hexane, cyclohexane, heptane, methylcyclohexane, ethylcyclohexane, octane, decane, dodecane, and tetradecane; an aromatic hydrocarbon such as benzene, toluene, ethylbenzene, xylene, trifluoromethylbenzene, and benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; a haloalkane such as dichloromethane, chloroform, 1,1-dichloroethane, 1,2-dichloroethane, 1,1,1-trichloroethane, and 1,1,2-trichloroethane; a carboxylic acid amide such as N,N-dimethylformamide and N,N-dimethylacetamide; and a sulfoxide such as dimethyl sulfoxide and diethyl sulfoxide. These may be used alone or in combination of two or more thereof.

By adjusting the solid content in the solution, the above-described conductive solution can have an appropriate viscosity for various coating methods such as spray coating and dip coating.

In addition, in a case where the above-described conductive solution contains the above-described conductive filler and the above-described silica particles (C), a lower limit value of a content of the silica particles (C) in the electrode portion main body 39 is, for example, 1 mass% or more, preferably 3 mass% or more, and more preferably 5 mass% or more, with respect to 100 mass% of the total amount of the silica particles (C) and the conductive filler. As a result, the mechanical strength of the electrode portion main body 39 can be improved. On the other hand, an upper limit value of the above-described content of the silica particles (C) in the electrode portion main body 39 is, for example, 20 mass% or less, preferably 15 mass% or less, and more preferably 10 mass% or less, with respect to 100 mass% of the total amount of the silica particles (C) and the conductive filler. As a result, a balance between the conductivity and the mechanical strength or flexibility in the electrode portion main body 39 can be achieved.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the electrode portion main body 39 can be suppressed.

### <Material of Conductive Contact Portion 33>

The conductive member of the conductive contact portion 33 is, for example, a paste containing an electrically conductive metal (so-called conductive paste). The electrically conductive metal includes one or more selected from the group consisting of copper, silver, gold, nickel, tin, lead, zinc, bismuth, antimony, and an alloy thereof. In particular, from the viewpoint of availability and conductivity, silver, silver chloride, or copper is preferable.

In a case where the conductive contact portion 33 is formed with the paste containing an electrically conductive metal, an apex portion of the protrusion portion 32 made of the rubber-like elastic body is dipped (dip-coated) in a paste-like conductive solution containing the electrically conductive metal. As a result, the conductive contact portion 33 is formed on the surface of the protrusion portion 32.

The conductive contact portion 33 as a conductive resin layer may be formed by applying the conductive solution containing the conductive filler and the solvent onto the protrusion portion 32. In this case, the solvent is based on the same material (silicone rubber) as the material of the protrusion portion 32, so that the adhesiveness of the conductive contact portion 33 (conductive resin layer) can be enhanced.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the conductive contact portion 33 can be suppressed.

As a result, it is possible to improve the hair-separating performance in a case where the brain wave measuring device 10 is mounted on the head 99. In addition, it is possible to sufficiently secure the contact area of the conductive contact portion 33 in a case where the brain wave measuring device 10 is mounted.

### <Structure of Signal Line Portion 34>

The electrode unit 30 is provided with a signal line portion 34 as a signal path connected to the conductive contact portion 33. Various wiring structures can be adopted for the signal line portion 34 as long as the signal line portion 34 is in a state of being conducted through the base portion 31 and the protrusion portion 32. Here, the signal line portion 34 is provided to pass through the inside of the protrusion portion 32 and the base portion 31 from the conductive contact portion 33 at the tip of the protrusion portion 32 and to be exposed on the base portion upper surface 37. A portion (here, an end portion 34a) of the signal line portion 34 which protrudes from the base portion upper surface 37 is interposed between the protruding snap button 35 (more specifically, a disk portion 35a described later) and the base portion upper surface 37, and electrical continuity with the protruding snap button 35 is ensured.

The lower-side tip of the signal line portion 34 may have any of a protruding structure, a substantially coplanar structure, or a buried structure with respect to a distal end portion of the protrusion portion 32 or the vicinity thereof, that is, the region where the conductive contact portion 33 is formed. From the viewpoint of connection stability with the conductive contact portion 33, a protruding structure may be used. A protruding portion at the distal end of the signal line portion 34 is partially or entirely covered with the conductive contact portion 33. For the protruding structure at the distal end of the signal line portion 34, it is possible to adopt a structure with or without folds or a structure wrapped around the surface of the distal end portion of the protrusion portion 32.

As another wiring structure of the signal line portion 34, a structure provided on the surfaces of the protrusion portion 32 and the base portion 31 may be used, or a wiring structure in which a part is provided on the inside and a part is provided on the surface may be used. That is, a signal detected by the conductive contact portion 33 may be finally transmitted to the protruding snap button 35.

### <Material of Signal Line Portion 34>

As the signal line portion 34, known materials can be used. For example, the signal line portion 34 can be configured with conductive fiber. As the conductive fiber, it is possible to use one or more fibers selected from the group consisting of metal fiber, metal-coated fiber, carbon fiber, conductive polymer fiber, conductive polymer-coated fiber, and conductive paste-coated fiber. These may be used alone or in combination of two or more thereof.

The metal material of the metal fiber and the metal-coated fiber is not limited as long as it has conductivity. Examples of the metal material include copper, silver, gold, nickel, tin, lead, zinc, bismuth, antimony, stainless steel, aluminum, silver/silver chloride, and alloys of these. These may be used alone or in combination of two or more thereof. Among these, from the viewpoint of conductivity, silver can be used. In addition, it is preferable that the metal material does not include a metal such as chromium, which imposes a burden on the environment.

The fiber material of the metal-coated fiber, the conductive polymer-coated fiber, or the conductive paste-coated fiber described above is not particularly limited and may be any one of synthetic fiber, semisynthetic fiber, or natural fiber. Among these, for example, polyester, nylon, polyurethane, silk, or cotton is preferably used. These may be used alone or in combination of two or more thereof.

Examples of the above-described carbon fiber include a PAN-based carbon fiber and a pitch-based carbon fiber.

As the conductive polymer material of the conductive polymer fiber and the conductive polymer-coated fiber, for example, a mixture of a conductive polymer such as polythiophene, polypyrrole, polyaniline, polyacetylene, polyphenylene vinylene, polynaphthalene, or a derivative of these, and a binder resin, or an aqueous solution of a conductive polymer such as PEDOT-PSS ((3,4-ethylenedioxythiophene)-poly(styrene sulfonate)) is used.

The resin material contained in the conductive paste of the conductive paste-coated fiber is not particularly limited, and preferably has stretchability. For example, the resin material includes one or more materials selected from the group consisting of silicone rubber, urethane rubber, fluororubber, nitrile rubber, acrylic rubber, styrene rubber, chloroprene rubber, and ethylene propylene rubber. These may be used alone or in combination of two or more thereof.

A conductive filler in the conductive paste of the conductive paste-coated fiber described above is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

A metal forming the above-described conductive filler is not particularly limited. For example, the metal may include at least one or two or more among copper, silver, gold, nickel, tin, lead, zinc, bismuth, antimony, silver/silver chloride, and alloys thereof. Among these, from the viewpoint of high conductivity or high availability, silver or copper is preferable.

The signal line portion 34 may be formed of twisted yarn obtained by twisting a plurality of linear conductive fibers. As a result, disconnection of the signal line portion 34 during deformation can be suppressed.

In the present embodiment, the coating of the conductive fiber includes not only that the outer surface of the fiber material is covered with the conductive material, but also that gaps between fibers in twisted yarn obtained by twisting single fibers are impregnated with metal, the conductive polymer, or the conductive paste such that each of the single fibers forming the twisted yarn is coated individually.

A tensile elongation at break of the signal line portion 34 is, for example, 1% or more and 50% or less, preferably 1.5% or more and 45% or less. In such a numerical range, excessive deformation of the protrusion portion 32 can be suppressed while suppressing break during deformation.

### <Protruding Snap Button 35 and Recessed Snap Button 25>

The protruding snap button 35 is made of, for example, an electrically conductive metal, and has a disk-shaped disk portion 35a and a convex button-shaped button portion 35b which extends from the center of the upper surface of the disk portion 35a. As the electrically conductive metal, for example, stainless steel, a copper alloy, an aluminum alloy, brass, or the like can be used.

The disk portion 35a is attached to the base portion upper surface 37 of the base portion 31 with a conductive adhesive or the like. In this case, as described above, the end portion 34a of the signal line portion 34 is interposed between the disk portion 35a and the base portion upper surface 37, and thus the conduction with the protruding snap button 35 is ensured.

The button portion 35b is attached to be fitted into the recessed snap button 25 provided in the support member 20.

As in the protruding snap button 35, the recessed snap button 25 is made of the electrically conductive metal, and outputs the brain waves acquired by the electrode unit 30 to a brain wave display device or the like through a predetermined wiring structure (not shown in the drawings).

### <Support Member 20>

The support member 20 is configured to have a long strip-shaped film base material. The support member 20 may be formed of one type of film base material, or may be a composite member formed of a plurality of film base materials. In any case, the support member 20 is configured to have sufficient strength for attaching the electrode unit 30. That is, the support member 20 has sufficient strength not to be damaged in a case where the recessed snap button 25 is provided to attach the electrode unit 30. In addition, the support member 20 has non-stretchable physical properties from the viewpoint of pressing the plurality of electrode units 30 against the head 99 with an appropriate pressure, that is, applying a constant tension.

A vertical width (length in a depth direction) of the support member 20 depends on the size of the electrode unit 30 to be mounted, but for example, can be 5 mm to 50 mm.

A lateral width (length in the left-right direction) of the support member 20 depends on the size of the head 99 and the electrode positions, but for example, can be 200 mm to 400 mm.

A thickness of the support member 20 depends on the material, but for example, can be 0.01 mm to 5 mm. The lower limit of the thickness is preferably 0.05 mm or more and more preferably 0.1 mm or more. The upper limit of the thickness is preferably 2 mm or less and more preferably 1 mm or less. By setting the thickness of the support member 20 to be within the above-described range, the electrode unit 30 can be pressed with a constant tension. The support member 20 may be curved to the extent that the tension does not substantially change, depending on the shape of the head 99 and the state of the hair.

As a material of the support member 20 (film member), for example, a resin member, a metal member, or a glass film can be used. Examples of the resin member include polyimide resin-based resin films such as a polyimide resin film, a polyetherimide resin film, and a polyamideimide resin film; polyamide resin-based films such as a polyamide resin film; polyester resin-based films such as a polyester resin film; polyethylene terephthalate (PET) resin-based films; and polystyrene (PS) resin-based films. Among the above, from the viewpoint of improving flexibility, elastic modulus, and heat resistance, a polyimide resin-based film is particularly preferably used. As the metal member, for example, an aluminum foil or a copper foil can be used.

### <Physical Property Values and the like of Support Member 20>

Physical property values (Poisson's ratio, Young's modulus, maximum thickness, Young's modulus × thickness) of the support member 20 are defined, for example, as follows.

### (Poisson's ratio)

A Poisson's ratio of the support member 20 is 0.15 to 0.4. The lower limit of the Poisson's ratio is preferably 0.2 or more, and more preferably 0.25 or more. The upper limit of the Poisson's ratio is preferably 0.38 or less, and more preferably 0.35 or less.

By setting the Poisson's ratio of the support member 20 to be within the above-described range, the support member 20 is less likely to be deformed. That is, in a case where the brain wave measuring device 10 is mounted on the head 99, a force acts in a direction in which the support member 20 is extended in a state where the electrode unit 30 is pressed against the head 99. That is, a tension acts. In this case, in a case where the support member 20 is an elastic member such as rubber, the support member 20 may be improperly stretched, the tension of the electrode unit 30 on the head 99 may change, and the pressing force may be non-uniform. As a result, signal quality of the obtained brain waves may be deteriorated. However, by setting the Poisson's ratio of the support member 20 to be within the above-described range, the force with which the electrode unit 30 presses the head 99 can be controlled to be in a certain range, and thus the brain wave measurement can be performed stably.

### (Young's modulus)

A Young's modulus (elastic modulus) of the support member 20 is 0.4 GPa to 150 GPa. The lower limit value of the Young's modulus is preferably 3 GPa or more, and more preferably 5 GPa or more. The upper limit value thereof is preferably 140 GPa or less and more preferably 135 GPa or less.

By setting the Young's modulus of the support member 20 to be within the above-described range, the strength of the support member 20 can be maintained, and thus deformation can be prevented.

### (Young's modulus × Thickness)

The product of the Young's modulus (elastic modulus) and the thickness of the material constituting the film member is 0.4 to 9.1.

Even in a case where the material is easily deformable (that is, the material has a small Young's modulus), if the thickness is equal to or greater than a certain extent, the force with which the electrode unit 30 presses the head 99 can be controlled within a certain range without substantially deforming the material. In addition, in a case of a hard material (that is, a material having a large Young's modulus), in a case where the thickness is not reduced to some extent, the characteristic of causing the brain wave measuring device 10 to follow the shape of the head 99 is significantly deteriorated. Therefore, by setting the product of the Young's modulus (elastic modulus) and the thickness within the above-described range, the force with which the electrode unit 30 attached to the support member 20 presses the head 99 can be controlled within a certain range, and thus stable measurement of the brain waves can be achieved.

### <Attachment Portion 70>

The attachment portion 70 is attached to each of both ends of the support member 20 in the longitudinal direction (both ends in the lateral direction in FIGS. 1 and 2), and is provided between a part (here, an ear) different from the measured site of the subject and the support member 20 to press the electrode unit 30 against the head 99 with the predetermined electrode pressing force F.

Specifically, the attachment portion 70 includes an adjustment unit 50, a tension display unit 60, an ear attachment portion 40, a non-stretchable cord 55 that connects the adjustment unit 50 and the tension display unit 60, and a cord 45 that connects the tension display unit 60 and the ear attachment portion 40.

The ear attachment portion 40 is mounted on the ear of the subject. The cord 45 is attached to the ear attachment portion 40, and is detachably attached to a second hook 64 of the tension display unit 60 by a ring 46 provided at an end portion of the cord 45. In the present embodiment, the ear attachment portion 40 is mounted such that the cord 45 is wound from below the ear.

The adjustment unit 50 is attached to each of both ends of the support member 20. The adjustment unit 50 includes a plate-shaped member 52 that is attached to the support member 20, a locking portion 51 for fixing a length of the plate-shaped member 52, a cord 55 that is attached to an end portion of the plate-shaped member 52 on the tension display unit 60 side, and a ring 56 that is provided at an end portion of the cord 55 on the tension display unit 60 side. A first hook 63 of the tension display unit 60 is detachably attached to the ring 56.

The plate-shaped member 52 has a long strip shape in which a plurality of teeth are arranged in a row. The locking portion 51 is provided with an opening in which a pawl is formed, and the plate-shaped member 52 is inserted into the opening and locked at a desired position. In addition, the locking portion 51 is provided with a release portion which releases the locked state in conjunction with the pawl. A material of the adjustment unit 50 is not particularly limited, and various plastics can be used. From the viewpoints of physical properties, processability, cost, and the like, Nylon 66 can be preferably used.

### <Tension Display Unit 60>

The tension display unit 60 is attachably and detachably provided between the adjustment unit 50 and the ear attachment portion 40, and displays whether or not a tension acting on both ends thereof is within an appropriate range. That is, it is possible to determine whether or not the tension T1 applied to the end portion of the support member 20 (film base material) is within an appropriate predetermined range in the brain wave measurement.

The tension display unit 60 includes a cylindrical housing 66, a spring 61 accommodated inside the housing 66, and an indicator 65, a scale 67, and an index 68 that indicate an extended state of the spring 61.

The housing 66 is configured to be able to determine at least a position of the indicator 65. In the present embodiment, the housing 66 is transparent such that an internal state is recognizable. One end portion (here, an end portion on the adjustment unit 50 side) of the housing 66 is closed, and the other end portion (here, an end portion on the ear attachment portion 40 side) is an opening. The first hook 63 is provided at an upper end portion of the housing 66, and is detachably attached to the ring 56 provided at a distal end of the cord 55 of the adjustment unit 50.

The spring 61 is, for example, a coil spring. One end portion (end portion on the adjustment unit 50 side) of the spring 61 is fixed to a closed end portion (end portion on the adjustment unit 50 side) of the housing 66.

A non-stretchable wire 62 is attached to the other end portion (end portion on the ear attachment portion 40 side) of the spring 61, and extends from an end portion of the housing 66 on the opening side to the outside. The second hook 64 is provided at a distal end of the wire 62. The second hook 64 is detachably attached to the ring 46 provided at an end portion of the cord 45 of the ear attachment portion 40.

The indicator 65 is provided at an attachment portion of the spring 61 and the wire 62. The indicator 65 indicates an extension of the spring 61. The scale 67 and the index 68 are provided on a peripheral surface of the housing 66. The scale 67 is provided with a plurality of horizontal lines at a constant interval. The index 68 is provided with numerical markings that allow the tension to be determined. The indicator 65, the scale 67, and the index 68 function as a tension appropriateness display unit that displays whether or not the tension is within an appropriate range in a recognizable manner.

It is also assumed that an appropriate tension varies depending on the attribute of the subject (for example, age, gender, and state of head hair). Therefore, a plurality of types of tension display units 60 having different strengths of the spring 61, ranges of the scale 67 and the index 68, and the like may be prepared, and an appropriate tension display unit 60 may be selected according to the subject.

As described above, according to the present embodiment, in the brain wave measuring device 10, the electrode unit 30 is fixed to the support member 20 configured with the film base material, and a polygonal shape is formed with the electrode units 30 attached only to the required portion as the vertices. Therefore, followability of the electrode unit 30 to the head shape can be improved. In addition, since the mounting state of the brain wave measuring device 10 is modeled by the capstan equation, the electrode pressing force F by the electrode unit 30 can be appropriately grasped.

In addition, by providing the adjustment unit 50 and the tension display unit 60 on the attachment portion 70, the tension acting on the support member 20 can be adjusted within an appropriate range. In addition, by attachably and detachably providing the tension display unit 60, an appropriate tension display unit 60 can be selected and used according to the attribute of the subject, and stable brain wave measurement can be realized.

Specifically, in a case where all of the support member 20 and the attachment portion 70 are non-stretchable members, in a case where the length is changed by the adjustment unit 50, the tension is directly changed. Since the appropriate range of the force with which the electrode unit 30 is pressed against the scalp is narrow, that is, the appropriate range of the adjustment unit 50 is also narrow, there is a tendency that the adjustment is difficult. More specifically, since the deformation and stretchability of the ear and the scalp are present, there is an effect of slightly widening the appropriate range of the adjustment unit 50, but the adjustment is still difficult, and the adjustment range between the state where the tension is small and the state is loose and the state where the tension is large and the subject feels pain is narrow, and it is difficult to mount the brain wave measuring device 10 by pressing the electrode unit 30 with a sufficient force without causing discomfort to the subject. On the other hand, in a case where the tension display unit 60 is provided, that is, in a case where a stretchable member is included, the deformation of the elastic body such as a more stretchable spring is added in addition to the deformation and stretchability of the ear and the scalp described above, so that the appropriate range of the adjustment unit 50 can be significantly widened, and it is easy to mount the brain wave measuring device 10 in an appropriate state.

### <Second Embodiment>

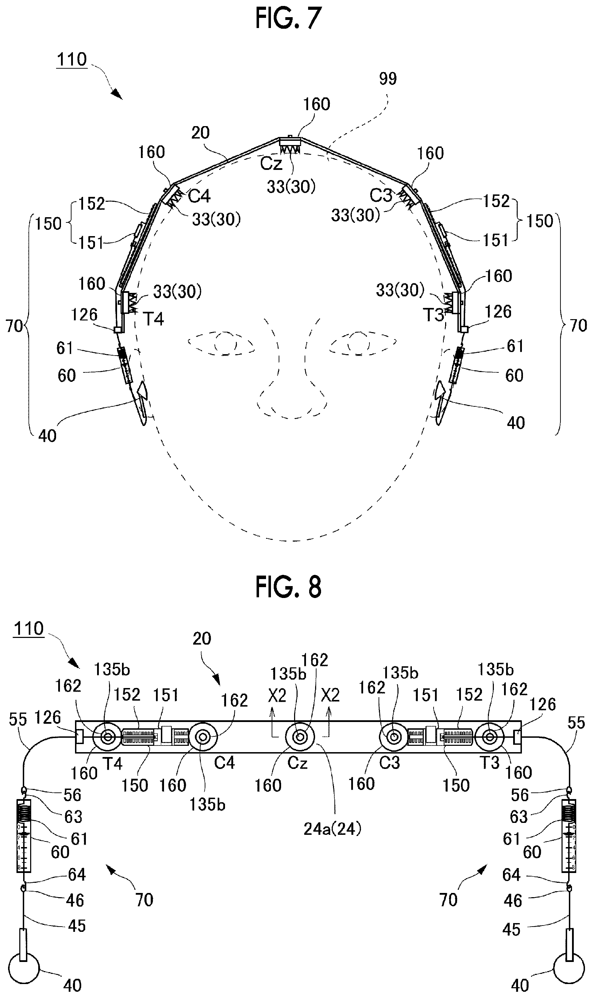

The second embodiment will be described with reference to FIGS. 7 to 10B. The present embodiment is different from the first embodiment in that (1) a mounting structure of the electrode unit 30 on the support member 20 is a screw fitting structure, (2) an electrode attachment portion 160 is provided instead of the recessed snap button 25 as a structure for attaching the electrode unit 30 in accordance with the adoption of the screw fitting structure, and (3) a structure and an installation position of the adjustment unit 150 are different. Hereinafter, the points different from the first embodiment will be mainly described, and the same configurations will be denoted by the same reference numerals as appropriate and the description thereof will not be repeated.

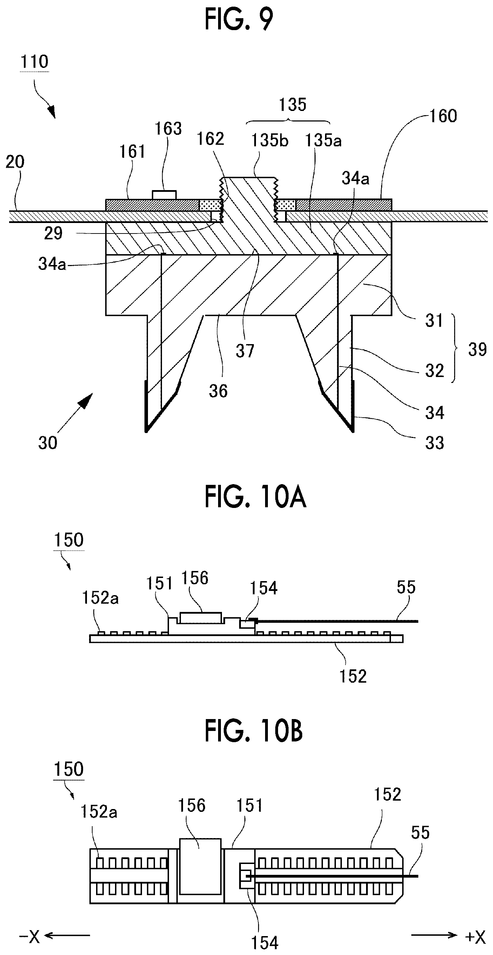

FIG. 7 is a schematic view of a state where a brain wave measuring device 110 is mounted on a head 99 of a person, as viewed from the front. FIG. 8 is a plan view showing the brain wave measuring device 110 in a state where the brain wave measuring device 110 is not mounted on the head 99. FIG. 9 is a cross-sectional view showing a state where the electrode unit 30 is attached to the electrode attachment portion 160 of the support member 20.

### <Electrode Attachment Portion 160>

The support member 20 is provided with the electrode attachment portion 160 for attaching the electrode unit 30. The electrode attachment portion 160 has an attachment portion main body 161 which is circular in top view and a female screw 162 which is provided at the center of the attachment portion main body 161.

An outer diameter of the attachment portion main body 161 is set to be substantially the same as the outer diameter of the electrode unit 30. A hard insulating substrate is provided on the upper surface of the attachment portion main body 161, and a circuit unit 163 (pre-amplifier) for primarily amplifying the brain waves acquired by the electrode unit 30 is mounted on the substrate. An upper surface of the attachment portion main body 161 is covered with an insulating cover member as necessary.

The female screw 162 made of a conductive member is attached to the center of the attachment portion main body 161. A male screw connection terminal 135 (protruding portion 135b) of the electrode unit 30 is screwed into the female screw 162.

### <Male Screw Connection Terminal 135>

On the upper surface of the electrode unit 30, instead of the protruding snap button 35 of the first embodiment, a male screw-type connection terminal 135 consisting of a conductive member is provided.

The male screw connection terminal 135 has a disk-shaped disk portion 135a and a protruding portion 135b which extends from a center of an upper surface of the disk portion 135a. The protruding portion 135b is formed in a cylindrical shape, and a male screw is formed on a peripheral surface thereof. The female screw 162 is connected to the circuit unit 163.

The electrode unit 30 is attached to the support member 20 by screwing the female screw 162 and the male screw connection terminal 135 (that is, the protruding portion 135b) to each other. The brain wave signal acquired by the electrode unit 30 is transmitted from the male screw connection terminal 135 to the circuit unit 163 through the female screw 162. The brain wave signal which has been subjected to primary amplification by the circuit unit 163 is output to a brain wave display device or the like through a signal path (not shown in the drawings).

By adopting the screw joining as the attachment aspect of the electrode unit 30 and the support member 20, the attachment portion can be stabilized and generation of noise can be suppressed.

### <Adjustment Unit 150>

FIGS. 10A and 10B show the adjustment unit 150. FIG. 10A is a side view, and FIG. 10B is a plan view.

The adjustment unit 150 is sewn to the support member 20 between the electrode position C3 and the electrode position T3 and between the electrode position C4 and the electrode position T4.

The adjustment unit 150 includes a plate-shaped member 152 which is attached to the support member 20, and a locking portion 151 which slides on the plate-shaped member 152 and fixes the plate-shaped member 152 at a desired position. The locking portion 151 is provided with a cord attachment portion 154 to which the cord 55 is attached.

Convex cord guide portions 126 are provided at both ends of the support member 20 in the longitudinal direction. The cord guide portion 126 is provided with an opening communicating in the left-right direction, and the cord 55 is inserted through the opening. As a result, the cord 55 always passes through both end portions of a base material 21, and thus the mounting state of the brain wave measuring device 10 can be made appropriate.

The plate-shaped member 152 is a rail-like long strip-shaped member in which a plurality of protruding portions 152a are arranged in a row. The locking portion 151 is slidably fitted to a rail formed by the protruding portions 152a. The locking portion 151 has a locking mechanism 156 holding in a non-slidable manner. The non-slidable state is released by a predetermined operation (for example, an operation of pushing in the horizontal direction) with respect to the locking mechanism 156.

By sliding the locking portion 151 on the plate-shaped member 152, the distance between the tension display unit 60 (that is, the ear attachment portion 40) attached to the cord 55 and the support member 20, that is, the tension can be adjusted.

### <Third Embodiment>

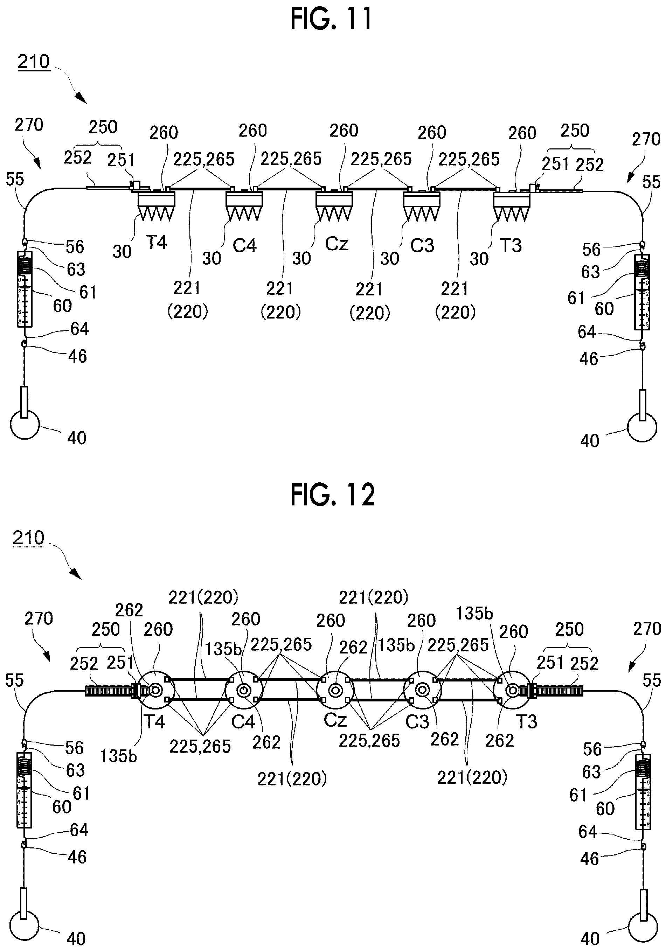

The third embodiment will be described with reference to FIGS. 11 to 14. The present embodiment is different from the first and second embodiments in that (1) a support member 220 is a linear member 221 and (2) the linear member 221 is configured to be detachable. Hereinafter, the points different from the first and second embodiments will be mainly described.

FIG. 11 is a plan view of a brain wave measuring device 210 according to the present embodiment. FIG. 12 is a front view of the brain wave measuring device 210. FIGS. 11 and 12 show the brain wave measuring device 210 in a state where the brain wave measuring device 210 is not mounted on the head 99. FIG. 13 is a plan view focusing on an attachment aspect of the support member 220 for two electrode units 30 at electrode positions Cz and C3. FIG. 14 is a cross-sectional view of the electrode unit 30 in a state of being attached to an electrode attachment portion 260.

The electrode unit 30 is attached to the electrode attachment portion 260 having the same configuration as the electrode attachment portion 160 shown in the second embodiment, and is supported by the support member 220. The support member 220 is provided with two parallel linear members 221 which are provided between adjacent electrode units 30 (that is, the electrode attachment portions 260). In addition, an attachment portion 270 is attached to each of end portion sides of the electrode attachment portions 260 (electrode positions T3 and T4) at right and left end portions.

### <Electrode Attachment Portion 260>

As in the second embodiment, the electrode attachment portion 260 has an attachment portion main body 261 which is circular in top view, a female screw 262 which is provided at the center of the attachment portion main body 261, and a circuit unit 263 which performs primary amplification on the acquired brain waves.

### <Connector 225>

The electrode attachment portion 260 has a plurality of connectors 225 for attaching the support member 220 (linear member 221).

On the upper surface of three of the electrode attachment portions 260 in the center (that is, electrode positions Cz, C3, and C4), two connectors 265 are provided on the right side in the drawing at the same position in the left-right direction on the front side and the back side, and two connectors 265 are provided on the left side in the drawing at the same position in the left-right direction on the front side and the back side.

The two connectors 265 on the right side each have an opening facing the right side, and are configured such that the connector 225 provided on the linear member 221 is insertable thereinto and removable therefrom. The two connectors 265 on the left side each have an opening facing the left side, and are insertable into and removable from the connector 225 provided on the linear member 221.

On the upper surface of the electrode attachment portion 260 at the right end (electrode position T3), two connectors 265 are arranged on the left side in the drawing on the front side and the back side, and an attachment portion 270 (adjustment unit 250) is provided near the center on the right side. The two connectors 265 each have an opening facing the left side, and are insertable into and removable from the connector 225 provided on the linear member 221.

On the upper surface of the electrode attachment portion 260 at the left end (electrode position T4), two connectors 265 are arranged on the right side in the drawing, and an attachment portion 270 (adjustment unit 250) is provided near the center on the left side. The two connectors 265 each have an opening facing the right side, and are insertable into and removable from the connector 225 provided on the linear member 221.

### <Attachment Portion 270>

The attachment portion 270 includes the ear attachment portion 40, the adjustment unit 250, the tension display unit 60, and the cord 55, as in the attachment portion 70 of the second embodiment. In the present embodiment, an attachment position of the adjustment unit 250 is the electrode attachment portion 260.

The adjustment unit 250 has a locking portion 251 attached to the electrode attachment portion 260 and a plate-shaped member 252 fixed in position by the locking portion 251. The cord 55 is attached to the plate-shaped member 252.

By sliding the plate-shaped member 252, the distance between the tension display unit 60 (that is, the ear attachment portion 40) attached to the cord 55 and the electrode attachment portion 260, that is, the tension can be adjusted.

### <Support Member 220>

The support member 220 has a linear member 221 and connectors 225 provided at both ends of the linear member 221.

The linear member 221 is, for example, a member such as a cord, a wire, or a cable, and is a member that can be freely deformed along at least the shape of the head 99. As the linear member 221, for example, an electric wire cable (a multi-core cable or a flat cable) can be used. A length of the linear member 221 is set according to the brain wave measurement position. A thickness of the linear member 221 may be set such that the linear member 221 has flexibility that can appropriately follow the head shape and sufficient strength that does not break even in a case where the brain wave measuring device 210 is mounted on the head 99 to measure the brain waves.

The connectors 225 at both ends of the linear member 221 are attached to the connectors 265 of the electrode attachment portion 260, thereby connecting the adjacent electrode attachment portions 260 (that is, the electrode units 30).

By using the electric wire cable as the linear member 221, the acquired brain wave signal can be aggregated at a predetermined external output terminal (not shown in the drawings). In addition, by using two linear members 221 parallel to each other between the adjacent electrode units 30, the posture of the electrode units 30 in contact with the head 99 can be stabilized. In addition, since the linear member 221 is detachable from the electrode attachment portion 260, it is possible to replace the linear member 221 or the electrode attachment portion 260 in a case where a part of the linear member 221 or the electrode attachment portion 260 is damaged or the like. In addition, by preparing linear members 221 having different lengths, an inter-electrode distance, that is, an optimum electrode position corresponding to the size of the head 99 of the subject can be set. The support member 220 may be fixed to the electrode attachment portion 260 by soldering or the like.

### <Summary of Embodiments>

The features of the embodiment are summarized as follows.
(1) A brain wave measuring device 10 including:
   a support member 20 which is disposed along a head 99 of a subject;
   an electrode unit 30 which is attached to the support member 20 and acquires a brain wave signal by coming into contact with a measured site of the subject; and
   an assistance member (attachment portion 70) which assists in positioning the support member 20 on the head 99,
   in which the support member 20 is configured with a non-stretchable member having a ribbon shape or a linear shape, and
   an elastic body (spring 61) having stretchability is provided between the support member 20 and an attachment portion to a human body (ear).
(2) The brain wave measuring device 10 according to (1),
   in which the elastic body (spring 61) is a tension display unit (tension display unit 60) that displays a tension acting on the support member 20 in a recognizable manner.
(3) The brain wave measuring device 10 according to (1) or (2), further including:
   a tension appropriateness display unit (indicator 65, scale 67, and index 68) that displays whether or not the tension is within an appropriate range in a recognizable manner.
(4) The brain wave measuring device 10 according to any one of (1) to (3),
   in which, in a case where the support member 20 is configured with a member having a ribbon shape, the support member 20 has a film member that is provided between adjacent electrode units 30.
(5) The brain wave measuring device 10 according to (4),
   in which a Poisson's ratio of a material forming the film member is 0.15 to 0.4.
(6) The brain wave measuring device 10 according to (4),
   in which a product of an elastic modulus and a thickness of a material forming the film member is 0.4 to 9.1.
(7) The brain wave measuring device 10 according to any one of (1) to (3),
   in which in a case where the support member 20 is configured with a member having a linear shape, the member having a linear shape is provided between electrode units 30.
(8) The brain wave measuring device 10 according to (7),
   in which the member having the linear shape includes at least two members that extend to be spaced apart from each other, and the electrode unit 30 is provided between the two members.
(9) The brain wave measuring device 10 according to any one of (1) to (8),
   in which the electrode unit 30 includes a base portion 31, a plurality of protrusions (protrusion portions 32) provided on the base portion 31, and an electrode portion (conductive contact portion 33) that is provided on the protruding portions (protrusion portions 32) and that comes into contact with the head 99.
(10) The brain wave measuring device 10 according to (9),
   in which the protruding portions (protrusion portions 32) are elastic members.
(11) The brain wave measuring device 10 according to any one of (1) to (10),
   in which the support member (attachment portion 70) is provided at an end portion of the support member 20, is attached to an ear or a jaw, and supports disposition of the support member 20 on the head 99.
(12) A brain wave measuring method including:
   mounting the brain wave measuring device 10 according to any one of (1) to (11) on a head of a subject to measure a brain wave.

The embodiments of the present invention have been described above, but these are examples of the present invention and various configurations other than the above can be adopted.

### [EXAMPLES]

Hereinafter, the present embodiments will be described in detail with reference to Examples. The present embodiments are not limited to the description of Examples.

The following examples correspond to the first and second embodiments, and "Verification of support member (film base material)" and "Verification of modeling based on capstan equation" are confirmed.

### <<Verification of Support Member (Film Base Material)>>

Seven samples of Examples 1 to 7 were evaluated as to whether or not they were appropriate as the support member 20.

The evaluation standards were in three stages as follows.
Evaluation A ... Deviation of the electrode position was small (5 mm or less) in a case where a constant tension (0.6 N) was applied.
Evaluation B ... Deviation of the electrode position was within an allowable range (10 mm or less) in a case where a constant tension (0.6 N) was applied.
Evaluation C ... Deviation of the electrode position was out of an allowable range (more than 10 mm) in a case where a constant tension (0.6 N) was applied.

Materials of each sample of Examples 1 to 7 are as follows. In addition, Table 1 shows each physical property (Poisson's ratio, Young's modulus, maximum thickness, Young's modulus × thickness).

| | |
|---|---|
| Example 1 | Polyethylene terephthalate (PET) resin |
| Example 2 | Polyimide (PI) resin |
| Example 3 | Aluminum foil |
| Example 4 | Copper foil |
| Example 5 | Polystyrene (PS) resin |
| Example 6 | Polyethylene (PE) resin |
| Example 7 | Glass film |

In Examples 1 to 6, the deviation of the electrode position in a case where a constant tension was applied was small, and thus the brain wave acquisition could be stably performed at the target position.

In Example 7, the Young's modulus of the support member was smaller than that of Examples 1 to 6, and the thickness was increased as compared with other samples in order to suppress deformation, but the deviation of the electrode in a case where a constant tension was applied was slightly large.

**[Table 1]**

| | Material | Poisson's ratio | Young's modulus [GPa] | Maximum thickness [mm] | Young's modulus × Thickness [GPa × mm] | Evaluation |
|---|---|---|---|---|---|---|
| Example 1 | PET | 0.25 to 0.4 | 5 | 0.5 | 2.5 | A |
| Example 2 | PI | 0.29 to 0.30 | 3.4 | 0.225 | 0.765 | A |
| Example 3 | Aluminum foil | 0.345 | 70.3 | 0.05 | 3.515 | A |
| Example 4 | Copper foil | 0.343 | 129.8 | 0.07 | 9.086 | A |
| Example 5 | PS | 0.34 to 0.38 | 2.7 to 4.2 | 0.2 | 1.08 to 2.1 | A |
| Example 6 | Glass film | 0.2 | 73 | 0.03 | 2.19 | A |
| Example 7 | PE | 0.26 | 0.4 to 1.3 | 1 | 0.4 to 1.3 | B |

### <<Verification of Modeling based on Capstan Equation>>

The brain wave measuring device described in the embodiment was mounted on a head model, the electrode pressing force F was measured using a pressure sensor disposed on the surface of the head model, and the measured value was compared with a calculated value obtained by calculation from the model based on the capstan equation.

Specific specifications of the brain wave measuring device 10 are as follows.
Brain wave measurement positions: five locations of T3, C3, Cz, C4, and T4
Electrode unit:
   Base portion diameter of 10 mm
   Thickness of base portion of 5 mm
   Height of protrusion portion of 5 mm
   Number of protrusion portions of 7
   Distance between electrodes of 70 mm
Support member (film base material):
   PI resin
   Poisson's ratio of 0.3
   Young's modulus of 3.4 GPa
   Maximum thickness of 0.225 mm

Instead of fixing the ear attachment portion to the ear, weights of 200 g each were attached to both ends to simulate the mounting state of the brain wave measuring device.

FIG. 15 shows a graph of theoretical values (calculated values) and measured values of the electrode pressing force F at the five locations of T3, C3, Cz, C4, and T4. As can be seen from the drawing, it can be confirmed that the theoretical values and the measured values substantially match, and thus the modeling is appropriate.

This application claims priority based on Japanese Patent Application No. 2023-180770 filed on October 20, 2023, the entire disclosure of which is incorporated herein by reference.

### REFERENCE SIGNS LIST

10, 110, 210 brain wave measuring device
20 support member (film base material)
25 recessed snap button
30 electrode unit
31 base portion
32 protrusion portion (protruding portion)
33 conductive contact portion (electrode portion)
34 signal line portion
35 protruding snap button
40 ear attachment portion
50, 150 adjustment unit
60 tension display unit
61 spring
62 wire
63 first hook
64 second hook
65 indicator
66 housing
67 scale
68 index
70 attachment portion
135 male screw connection terminal
160, 260 electrode attachment portion
161, 261 attachment portion main body
162, 262 female screw
220 support member
221 linear member
225, 265 connector

## Claims

1. A brain wave measuring device comprising:
a support member which is disposed along a head of a subject;
an electrode unit which is attached to the support member and acquires a brain wave signal by coming into contact with a measured site of the subject; and
an assistance member which assists in positioning the support member on the head,
wherein the support member is configured with a non-stretchable member having a ribbon shape or a linear shape, and
an elastic body having stretchability is provided between the support member and an attachment portion to a human body.

2. The brain wave measuring device according to Claim 1,
wherein the elastic body is a tension display unit that displays a tension acting on the support member in a recognizable manner.

3. The brain wave measuring device according to Claim 2, further comprising:
a tension appropriateness display unit that displays whether or not the tension is within an appropriate range in a recognizable manner.

4. The brain wave measuring device according to Claim 1 or 2,
wherein, in a case where the support member is configured with a member having a ribbon shape, the support member has a film member which is provided between adjacent electrode units.

5. The brain wave measuring device according to Claim 4,
wherein a Poisson's ratio of a material forming the film member is equal to or more than 0.15 and equal to or less than 0.4.

6. The brain wave measuring device according to Claim 4,
wherein a product of an elastic modulus and a thickness of a material forming the film member is equal to or more than 0.4 and equal to or less than 9.1.

7. The brain wave measuring device according to Claim 1 or 2,
wherein in a case where the support member is configured with a member having a linear shape, the member having a linear shape is provided between electrode units.

8. The brain wave measuring device according to Claim 7,
wherein the member having the linear shape includes at least two members that extend to be spaced apart from each other, and the electrode units are provided between the two members.

9. The brain wave measuring device according to Claim 1 or 2,
wherein the electrode unit includes a base portion, a plurality of protruding portions provided on the base portion, and an electrode portion that is provided on the protruding portions and that comes into contact with the head.

10. The brain wave measuring device according to Claim 9,
wherein the protruding portions are elastic members.

11. The brain wave measuring device according to Claim 1 or 2,
wherein the assistance member is provided at an end portion of the support member, is attached to an ear or a jaw, and assists in positioning the support member along a shape of the head.

12. A brain wave measuring method comprising:
mounting the brain wave measuring device according to Claim 1 or 2 on a head of a subject to measure a brain wave.
